# EUROPÄISCHE PATENTSCHRIFT

(11) **EP 2 964 662 B1**
(45) Veröffentlichungstag und Bekanntmachung des Hinweises auf die Patenterteilung: **22.08.2018**
(21) Anmeldenummer: 14707364.7
(22) Anmeldetag: 26.02.2014
(51) Int. Cl.: C07K 1/14, C07K 16/00, B01D 17/02, B01D 21/26

(54) **PROTEINREINIGUNG MITTELS WÄSSRIGER ZWEI-PHASEN-ZENTRIFUGAL-EXTRAKTION**
PROTEIN PURIFICATION BY MEANS OF AQUEOUS TWO PHASE CENTRIFUGAL EXTRACTION
PURIFICATION DE PROTÉINES PAR EXTRACTION CENTRIFUGE AQUEUSE À DEUX PHASES

(30) Priorität: 08.03.2013 EP 13158285
(43) Veröffentlichungstag der Anmeldung: 13.01.2016
(73) Patentinhaber: Boehringer Ingelheim International GmbH, 55216 Ingelheim am Rhein (DE)
(72) Erfinder: RICHTER, Michael, 55216 Ingelheim Am Rhein (DE); DIETERLE, Michael, 55216 Ingelheim Am Rhein (DE); RUDOLPH, Frederik, 55216 Ingelheim Am Rhein (DE)
(74) Vertreter: Simon, Elke Anna Maria
(86) Internationale Anmeldenummer: PCT/EP2014/053768
(87) Internationale Veröffentlichungsnummer: WO 2014/135420

(56) Entgegenhaltungen:
- WO-A1-2010/062244
- ANDRES VEIDE ET AL: "A Process for Large-Scale Isolation of Ã -Galactosidase from E. coli in an Aqueous Two-Phase System", BIOTECHNOLOGY AND BIOENGINEERING, WILEY & SONS, HOBOKEN, NJ, US, Bd. 25, Nr. 7, 1. Juli 1983 (1983-07-01), Seiten 1789-1800, XP001312741, ISSN: 0006-3592
- FOLKE TJERNELD ET AL: "Affinity liquid-liquid extraction of lactate dehydrogenase on a large scale", BIOTECHNOLOGY AND BIOENGINEERING, Bd. 30, Nr. 7, 1. November 1987 (1987-11-01), Seiten 809-816, XP055070668, ISSN: 0006-3592, DOI: 10.1002/bit.260300702
- ROSA P A J ET AL: "Application of aqueous two-phase systems to antibody purification: A multi-stage approach", JOURNAL OF BIOTECHNOLOGY, ELSEVIER SCIENCE PUBLISHERS, AMSTERDAM, NL, Bd. 139, Nr. 4, 23. Februar 2009 (2009-02-23), Seiten 306-313, XP025987456, ISSN: 0168-1656, DOI: 10.1016/J.JBIOTEC.2009.01.001 [gefunden am 2009-01-17]
- ROSA P A J ET AL: "Downstream processing of antibodies: Single-stage versus multi-stage aqueous two-phase extraction", JOURNAL OF CHROMATOGRAPHY, ELSEVIER SCIENCE PUBLISHERS B.V, NL, Bd. 1216, Nr. 50, 11. Dezember 2009 (2009-12-11), Seiten 8741-8749, XP026766451, ISSN: 0021-9673, DOI: 10.1016/J.CHROMA.2009.02.024 [gefunden am 2009-02-13]
- JOSEFINE PERSSON ET AL: "Evaluation of different primary recovery methods forE. coli-derived recombinant human growth hormone and compatibility with further down-stream purification", BIOTECHNOLOGY AND BIOENGINEERING, Bd. 90, Nr. 4, 20. Mai 2005 (2005-05-20), Seiten 442-451, XP055070670, ISSN: 0006-3592, DOI: 10.1002/bit.20434
- AZEVEDO A M ET AL: "Partitioning of human antibodies in polyethylene glycol-sodium citrate aqueous two-phase systems", SEPARATION AND PURIFICATION TECHNOLOGY, ELSEVIER SCIENCE, AMSTERDAM, NL, Bd. 65, Nr. 1, 2. Februar 2009 (2009-02-02), Seiten 14-21, XP025769313, ISSN: 1383-5866, DOI: 10.1016/J.SEPPUR.2007.12.010 [gefunden am 2007-12-27]
- ANDREWS B A ET AL: "Partitioning and purification of monoclonal antibodies in aqueous two-phase systems", BIOSEPARATION, KLUWER ACADEMIC PUBLISHERS, DORDRECHT, NL, Bd. 6, Nr. 5, 1. Januar 1996 (1996-01-01), Seiten 303-313, XP008091670, ISSN: 0923-179X
- CASCONE O ET AL: "Partitioning and purification of thaumatin in aqueous two-phase systems", ENZYME AND MICROBIAL TECHNOLOGY, STONEHAM, MA, US, Bd. 13, Nr. 8, 1. August 1991 (1991-08-01) , Seiten 629-635, XP023990159, ISSN: 0141-0229, DOI: 10.1016/0141-0229(91)90076-M [gefunden am 1991-08-01]
- DIMITRIS PLATIS ET AL: "Application of a PEG/salt aqueous two-phase partition system for the recovery of monoclonal antibodies from unclarified transgenic tobacco extract", BIOTECHNOLOGY JOURNAL, Bd. 4, Nr. 9, 25. September 2009 (2009-09-25), Seiten 1320-1327, XP055070866, ISSN: 1860-6768, DOI: 10.1002/biot.200800359
- LISONG NATHAN MAO ET AL: "Downstream antibody purification using aqueous two-phase extraction", BIOTECHNOLOGY PROGRESS,, Bd. 26, Nr. 6, 1. November 2010 (2010-11-01), Seiten 1662-1670, XP002638888, ISSN: 1520-6033, DOI: 10.1002/BTPR.477 [gefunden am 2010-06-29]
- ROSA ET AL: "Affinity partitioning of human antibodies in aqueous two-phase systems", JOURNAL OF CHROMATOGRAPHY, ELSEVIER SCIENCE PUBLISHERS B.V, NL, Bd. 1162, Nr. 1, 31. Juli 2007 (2007-07-31), Seiten 103-113, XP022182050, ISSN: 0021-9673, DOI: 10.1016/J.CHROMA.2007.03.067
- "CENTRIFUGE EXTRACTION AND SEPARATION", , 1. Januar 2006 (2006-01-01), XP055070959, Gefunden im Internet: URL:http://www.verdeanalitica.com.br/catal ago/kromaton_02.pdf [gefunden am 2013-07-11]

## Beschreibung

### HINTERGRUND DER ERFINDUNG

### TECHNISCHES GEBIET

Die vorliegende Erfindung betrifft Reinigungsverfahren für Proteine, insbesondere Immunglobuline.

### STAND DER TECHNIK

Biomoleküle wie Proteine, Polynukleotide, Polysaccharide und dergleichen gewinnen als Medikamente, als Diagnostika, als Zusatzstoffe in Nahrungsmitteln, Waschmitteln und dergleichen, als Forschungsreagenzien und für viele weitere Anwendungen zunehmend an kommerzieller Bedeutung. Der Bedarf an solchen Biomolekülen lässt sich - z.B. im Falle von Proteinen - in aller Regel nicht mehr durch Isolierung der Moleküle aus natürlichen Quellen befriedigen, sondern erfordert den Einsatz biotechnologischer Produktionsmethoden.

Die biotechnologische Herstellung von Proteinen beginnt typischerweise mit der Klonierung eines DNS-Fragmentes in einen geeigneten Expressionsvektor. Nach Transfektion des Expressionsvektors in geeignete prokaryontische oder eukaryontische Expressionszellen und anschließender Selektion transfizierter Zellen werden letztere in Fermentern kultiviert und das gewünschte Protein zur Expression gebracht. Anschließend erfolgt die Ernte der Zellen bzw. des Kulturüberstandes und die Aufarbeitung und Reinigung des darin enthaltenen Proteins.

Im Falle eukaryontischer Expressionssysteme, also bei Verwendung von Säugetierzellkulturen wie etwa CHO- oder NS0-Zellen, konnte in den zurückliegenden 15 Jahren eine deutliche Steigerung der beim Expressionsschritt erreichbaren Konzentration des gewünschten Proteins in den Zellkulturen bzw. Zellkulturüberständen erzielt werden (1). Im gleichen Zeitraum stieg die Bindekapazität von Chromatographiematerialien, die bei der anschließenden Aufreinigung der Proteine eingesetzt werden, im Vergleich nur geringfügig. Aus diesem Grund besteht ein dringender Bedarf an verbesserten, optimierten Aufreinigungsverfahren für Biomoleküle, insbesondere Proteine, die in großem, industriellen Maßstab durchgeführt werden können (2).

Im Falle von Biopharmazeutika, wie etwa als Medikamente eingesetzten Proteinen, z.B. therapeutischen Antikörpern, ist neben der Produktausbeute auch die Abtrennung von Verunreinigungen von großer Bedeutung. Hierbei können prozess- und produktabhängige Verunreinigungen unterschieden werden (3). Die prozessabhängigen Verunreinigungen beinhalten Wirtszellen sowie Komponenten der Wirtszelle wie z.B. Proteine (Wirtszellproteine, "Host cell proteins", HCP) und Nukleinsäuren. Diese stammen u.a. aus der Zellkultur (wie Medienbestandteile) oder aus der Aufarbeitung (wie etwa Salze oder abgelöste Chromatographie-Liganden). Produktabhängige Verunreinigungen sind molekulare Varianten des Produkts mit abweichenden Eigenschaften (4). Hierzu zählen verkürzte Formen wie Vorläufer und hydrolytische Abbauprodukte, aber auch modifizierte Formen, gebildet beispielsweise durch Desaminierungen, falsche Glykosylierungen oder falsch verknüpfte Disulfidbrücken. Ebenso zu den produktabhängigen Varianten zählen Polymere und Aggregate. Weitere Verunreinigungen sind Kontaminanten. Damit werden alle weiteren Materialien chemischer, biochemischer oder mikrobiologischer Natur bezeichnet, die nicht direkt zum Herstellungsprozess gehören. Kontaminanten sind z.B. Viren, die unerwünschter weise in Zellkulturen auftreten können.

Verunreinigungen führen im Falle von Biopharmazeutika zu Sicherheitsbedenken. Diese werden verstärkt, wenn - wie sehr häufig bei Biopharmazeutika - die Verabreichung der therapeutischen Proteine über Injektion oder Infusion direkt in die Blutbahn erfolgt. So können Wirtszellen und Wirtszellkomponenten zu allergischen Reaktionen oder immunopathologischen Effekten führen. Daneben können Verunreinigungen auch zu einer unerwünschten Immunogenität des verabreichten Proteins führen, also eine unerwünschte Immunreaktion des Patienten auf das Therapeutikum auslösen, bis hin zu einem lebensbedrohenden anaphylaktischen Schock. Daher besteht ein Bedarf an geeigneten Reinigungsprozessen, mit denen alle unerwünschten Substanzen auf ein unbedenkliches Maß abgereichert werden können.

Andererseits können auch im Falle von Biopharmazeutika wirtschaftliche Aspekte nicht ignoriert werden. So sollen die eingesetzten Produktions- und Reinigungsverfahren die Rentabilität des damit erzeugten biopharmazeutischen Produkts nicht gefährden.

Zur Aufarbeitung von Proteinen aus Zellkulturen werden in der Biopharmazie mehrere Prozessschritte angewendet. Als erstes werden die Wirtszellen mittels eines Separators aus dem Zellkultur getrennt. Danach werden erste Verunreinigungen und Trübungen sowie letzte Wirtszellen mittels einer Filterkaskade (Tiefenfilter und Klärfilter sowie Sterilfilter) befreit. Dabei bleiben geladene Moleküle wie DNA, HCP und Trübungen in den Filtermaterialien zurück (2). Dieser Zellkulturüberstand kann dann in weiteren Aufarbeitungsschritten mit Chromatographiesäulen und Filtern höchst rein isoliert werden.

Die Wirtszellernte ist jedoch zeit- und kostenaufwendig und gerade bei steigenden Produktkonzentrationen im Fermenter und hohen Produktmengen kommt es bei Filtrationen zu Limitierungen. Kritische Punkte sind hierbei: Beladungskapazitäten, Prozessdruck durch Trübungen und somit lange Prozesszeiten (5).

Als Aufarbeitungs- und Reinigungsschritt von Proteinen aus dem Zellkulturüberstand kommt neben Filtrations- und Fällungsschritten den (säulenchromatographischen) Verfahren eine ganz zentrale Bedeutung zu. So beinhaltet häufig gerade die Antikörper Anreicherung einen affinitätschromatographischen Reinigungsschritt. Dementsprechend sind heutzutage zahlreiche säulenchromatographische Verfahren und hierbei verwendbare Chromatographiematerialen bekannt.

Affinitätschromatographie-Matrices werden bei der industriellen Reinigung von verschiedenen Substanzen als stationäre Phase eingesetzt (6). Über immobilisierte Liganden lassen sich Substanzen spezifisch anreichern und reinigen, die eine gewisse Affinität zu dem jeweils verwendeten Liganden besitzen. Für die industrielle Aufreinigung von Antikörpern (Immunglobulinen), insbesondere die Reinigung monoklonaler Antikörper, wird häufig immobilisiertes Protein A als initialer Reinigungsschritt verwendet. Protein A ist ein Protein mit etwa 41 kDA aus *Staphylococcus aureus,* das mit hoher Affinität (10⁻⁸ M - 10⁻¹² M an humanem IgG) an die CH₂/CH₃-Domäne der Fc-Region von Immunglobulinen bindet. Bei der Protein A-Chromatographie binden Immunglobuline oder Fusionsproteine, die eine Protein A-bindende Fc-Region aufweisen, aus der mobilen Phase spezifisch an den Protein A-Liganden, der kovalent an einen Träger (z.B. Sepharose) gekoppelt ist. Protein A aus *Staphylococcus aureus* (wildtyp Protein A) sowie gentechnisch verändertes, rekombinantes Protein A (rec. Protein A) interagiert über nicht-kovalente Wechselwirkungen mit der konstanten Region (Fc-Fragment) der Antikörper (7). Diese spezifische Interaktion kann ausgenutzt werden um Verunreinigungen effizient von dem Antikörper abzutrennen. Durch eine Veränderung des pH-Werts kann die Interaktion zwischen Antikörper und Protein A-Liganden gezielt aufgelöst und die Antikörper von der stationären Phase freigesetzt bzw. eluiert werden.

Die Affinitätschromatographie ist jedoch kostenaufwendig und gerade bei steigenden Produktkonzentrationen im Fermenter und hohen Produktmengen kommt es bei chromatographischen Reinigungsprozessen zu Limitierungen. Kritische Punkte sind hierbei: Beladungskapazitäten, Zyklenzahl, Prozesszeiten, Poolvolumina und Puffermengen. Alternative Reinigungsverfahren sind daher für die Zukunft unerlässlich. Eine generelle Übersicht zu konventionellen Reinigungsstrategien, wie auch der Affinitätschromatographie und alternative Methoden der Affinitätschromatographie bieten folgende Artikel (8; 9)

Die Pharmaforschung und Entwicklung arbeitet seit einigen Jahren verstärkt an neuen Biopharmazeutika, welche keine Fc-Region besitzen (10). Für diese nicht Antikörper bzw. Proteine gibt es bisher wenig geeignete Chromatograhiematrices auf den Markt. Preise für optimierte Matrices (Beladungskapazität) sowie die Entwicklungszeit für Prozesse mit neuen Matrices werden ansteigen (11).

Die Fermentation ist ein volumenbezogener Prozess, d.h. eine Erhöhung der Produktkonzentration hat keinen direkten Einfluss auf den Prozess, da die gleichen Apparate, wie Fermentationsreaktoren, verwendet werden können. In den meisten konventionellen Proteinaufreinigungsprozessen werden adsorptions- und filtrationsbasierte Techniken eingesetzt. Diese arbeiten massenbezogen und müssen deshalb bei steigenden Produktkonzentrationen größere Produktmengen aufreinigen und können dabei an ihre Beladungsgrenzen stoßen. Da diese Techniken nicht beliebig nach oben skalierbar sind oder ihr Einsatz sehr kostenintensiv ist, wird die Nutzung alternativer Techniken zunehmend in Betracht gezogen (12).

Eine vielversprechende Alternative zu den bereits oben erwähnten adsorptions- und filtrationsbasierten Techniken ist die schonende Extraktion von Biomolekülen (Proteine, Nukleinsäuren, Zellen, etc.) mittels wässriger Zwei-Phasen-Systeme (engl. Aqueous Two Phase System, ATPS). ATPS besteht aus einer wässrigen Lösung mit zwei unmischbaren Polymeren, bzw. einem Polymer und einem anorganischen Salz. Nach der Mischung der oben genannten Komponenten kommt es zur Ausbildung von zwei Phasen im System. Aufgrund unterschiedlicher biophysikalischer Eigenschaften erfolgt eine Verteilung der Biomoleküle in eine der beiden Phasen. Die Aufkonzentrierung und Aufreinigung des Zielmoleküls kann so in einem Prozessschritt erreicht werden. Polyethylenglykol (PEG) ist eines der am häufigsten zur Phasenbildung verwendeten Polymere (19).

Das wässrige Zwei-Phasen-System wurde 1957 zum ersten Mal vorgestellt. Seitdem werden wässrige Zwei-Phasen-Systeme für die Aufreinigung einer Vielzahl von Proteinen aber auch Plasmid-DNA eingesetzt (13; 14; 15). Für eine höhere Reinheit des Zielmoleküls können mehrstufige ATPS-Extraktionen durchgeführt werden. So konnte z.B. die Reinheit von Immunoglobulin G von 43% nach einer einstufigen ATPS-Extraktion auf 93% mittels einer 4-stufigen ATPS-Extraktion verbessert werden (16).

Für einen industriellen Einsatz von ATPS fehlt bis zum heutigen Tag aber das nötige Prozessverständnis. Dafür müssen Prozessparameter evaluiert und Scale-up Daten erhoben werden sowie phasenbildende Komponenten näher charakterisiert werden.

Zur Trennung von wässrigen Zwei-Phasen-Systemen können Extraktionsapparaturen wie z.B. Bodenkolonnen oder Mischabsetzer (*Mixer Settler*) eingesetzt werden (17).

Die Bodenkolonne, die für die Extraktion von flüssigen Phasen verwendet werden, besteht aus zwei Abscheidern und einem zentralen zylindrischen Teil. Die Abscheider, die sich jeweils unten und oben an der Kolonne befinden, enthalten einen Ein- und Ausgange für die leichte bzw. schwere Phase. Ein Pulsationssystem, das sich an der Basis des zentralen Mittelteils befindet, ermöglicht eine gleichmäßige Verteilung und Durchmischung der beiden Phasen über den Kolonnenquerschnitt und somit Bedingungen für einen Stoffaustausch zwischen den beiden Phasen.

Die Mixer-Settler-Apparatur besteht aus einer Mixer- und einer Settlerkammer. In der Mixerkammer werden die beiden Phasen durch einen drehzahlgesteuerten Rührer angesaugt und dispergiert. Nach der Durchmischung wird die Flüssigkeit in die Settler-Kammer zur Absetzung der beiden Phasen überführt.

Eine weitere verfahrenstechnische Apparatur zur Trennung zweier nichtmischbarer Flüssigkeiten ist der Zentrifugalextraktor.

In einem Zentrifugalextraktor werden zwei nicht mischbare Flüssigkeiten mit unterschiedlichen Dichten in eine Pumpenkammer eingespeist. Die flüssig/flüssig-Mischung wird in den Zentrifugenbecher durch eine Pumpenturbine, die sich am Boden des rotierenden Bechers befindet, eingezogen. Die Flüssigkeiten werden durch Zentrifugalkraft, welche durch den rotierenden Becher entsteht, separiert. Die schwere Phase reichert sich im äußeren Bereich des Zentrifugenbechers an. Die leichte Phase reichert sich im inneren Bereich an. Die Position der flüssig/flüssig Interphase wird durch ein sogenanntes Schwere-Phasen-Wehr geregelt. Austauschbare Schwere-Phasen-Wehre mit unterschiedlichen Durchmessern bieten die Möglichkeit, unterschiedliche Dichte-Verhältnisse der Flüssigkeiten zu prozessieren. Die schwere Phase wird über eine stationäre Kammer aufgenommen und über einen Ablauf abgegeben. Die leichte Phase wird ebenfalls in einer separaten stationären Kammer aufgenommen und über einen weiteren Ablauf zur weiteren Prozessierung abgegeben.

Bei einem flüssig/flüssig-Zentrifugalextraktor können drei Prozessparameter eingestellt werden: der Beladungs- bzw. Pumpenfluss, die Rotationsgeschwindigkeit des Zentrifugenbechers sowie die Interphasenposition.

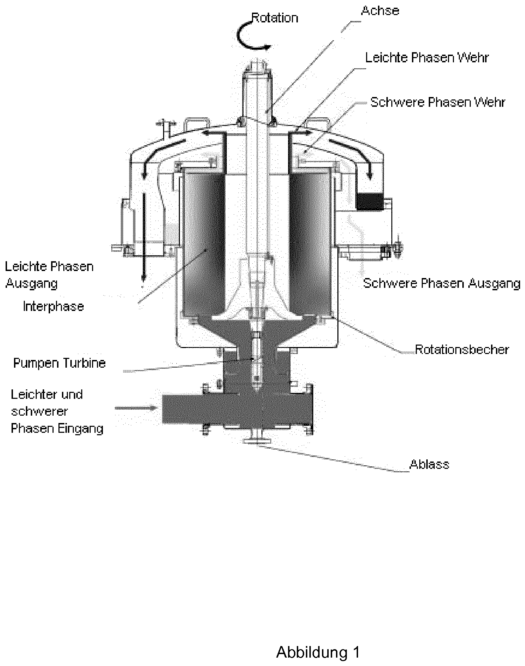

Abbildung 1 zeigt die schematische Darstellung eines Zentrifugalextraktors des Herstellers Rousselet-Robatel.

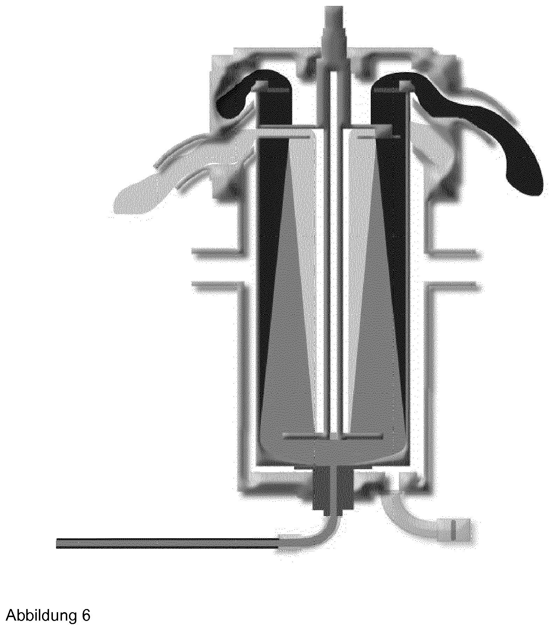

Abbildung 6 zeigt das Prinzip eines Zentrifugalextraktors nach dem "Direct Feed"-Prinzip (Quelle: Wikipedia/Vornefeld; http://commons.wikimedia.org/wiki/File:Sep-DF-7.jpg). Bei "Direct feed" erfolgt die Zuführung der gemischten Flüssigkeiten durch die Bodenplatte direkt in den Rotor und minimiert somit die Scherkräfte auf das Produkt. Die beiden Phasen werden kontinuierlich in eine schwere Phase und eine leichte Phase separiert.

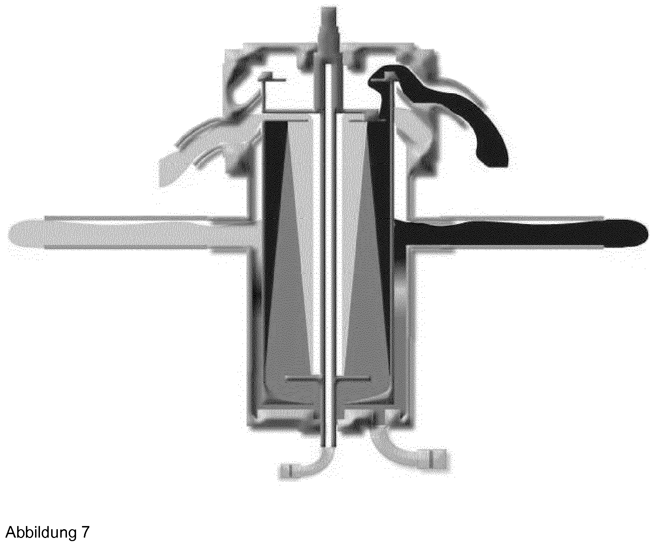

Abbildung 7 zeigt das Prinzip eines Zentrifugalextraktors nach dem "mix und sep"-Prinzip (Quelle: Wikipedia/Vornefeld, http://commons.wikimedia.org/wiki/File:Mixsep.jpg). Bei "mix & sep" werden zwei unmischbare Flüssigkeiten unterschiedlicher Dichte außerhalb des Rotors in Kontakt gebracht. Durch die im engen Spalt zwischen sich drehendem Rotor und statischer Gehäusewand entstehenden Scherkräfte werden sehr kleine Tropfen mit grosser Oberflächen für den idealen Stoffübergang von einer Phase in die andere Phase gebildet. Im Rotor werden anschließend beide Phasen voneinander getrennt.

### KURZE ZUSAMMENFASSUNG DER ERFINDUNG

Die vorliegende Erfindung ist eine Kombination aus Zellernte, Proteinseparation sowie der DNA Abreicherung aus Zellkulturen mit Hilfe von wässrigen Zwei-Phasen-Systemen und der Nutzung eines Zentrifugalextraktors. Bei der Erfindung wird vollständig auf den herkömmlichen Einsatz von Separator, Filtration und Chromatographie verzichtet.

Die hier beschriebene Erfindung bedient sich der wässrigen Zwei-PhasenTrennung (Extraktion) anstelle der Prozessschritte zur Separation und Filtration von Wirtszellen sowie der Affinitätschromatographie zur Reinigung von Biomolekülen. Zur Durchführung der Extraktion wird ein Zentrifugalextraktor benutzt. Dieser kann die Extraktionszeit bzw. eine Trennung der verwendeten Phasen Systeme in leichte und schwere Phase im Produktionsmaßstab von z.B. 12000L Zellkultur in wenigen Stunden realisieren. Eine Prozessierung des gleichen Volumens im oben beschriebenen Mixer Settler oder einer Bodenkolonne würde mehrere Tage dauern. Unter Benutzung eines Zentrifugalextraktors lassen sich wässrige Zwei-Phasen-Extraktionen im Vergleich zu Trennung mit alternativen Techniken wie der Bodenkolonne oder dem Mixer Settler, um das 50- 250 fache schneller prozessieren.

Mit Hilfe des erfindungsgemäßen Verfahrens lassen sich Proteine stabil und ohne Aggregation anreichern.

In einem Aspekt betrifft die Erfindung ein Verfahren zur selektiven Reinigung und Anreicherung von Immunglobulinen mittels eines wässrigen Zwei-Phasen-Systems mit den Schritten
a. Bereitstellen einer Zellkultur oder eines Zellkulturüberstandes, die das Zielprotein enthält;
b. Überführung der Zellkultur oder des Zellkulturüberstandes in ein wässriges Zweiphasensystem durch Zugabe von Polyethylenglykol mit einen Molekulargewicht zwischen 200 und 1000 g/mol, so dass das Polyethylenglykol in einer Konzentration von 18 bis 35 Gewichts-% vorliegt und eines Phosphatsalzes,;
c. Durchmischung des Zweiphasensystems zur Erzeugung einer Dispersion;
d. Abtrennung von schwerer und leichter Phase in einem Zentrifugalextraktor;
e. Gewinnung des Zielproteins aus der leichten Phase.

Unter "Reinigung und Anreicherung" ist dabei zu verstehen, dass unerwünschte Komponenten wie Zellen, DNA und Wirtsproteine abgereichert werden können und die Reinheit des Zielproteins durch das Verfahren zunimmt.

In einem anderen Aspekt betrifft die Erfindung ein Verfahren zur selektiven Reinigung und Anreicherung und zusätzlichen Ankonzentrierung von Immunglobulinen mittels eines wässrigen Zwei-Phasen-Systems mit den Schritten
a. Bereitstellen einer Zellkultur oder eines Zellkulturüberstandes, die das Zielprotein enthält;
b. Bereitstellen einer leichten Phase des wässriges Zweiphasensystems durch Zugabe von Polyethylenglykol mit einen Molekulargewicht zwischen 200 und 1000 g/mol und eines Phosphatsalzes, ;
c. Überführen der Zellkultur oder des Zellkulturüberstandes in die schwere Phase des wässriges Zweiphasensystem durchs Zugabe von Polyethylenglykol mit einen Molekulargewicht zwischen 200 und 1000 g/mol und eines Phosphatsalzes;
d. Separate Zuführung von leichter und schwerer Phase in die Mischkammer des Zentrifugalextraktors mit unterschiedlichen Flussraten, wobei das Verhältnis der Flussraten dem gewünschten Ankonzentrierungsverhältnis entspricht;
e. Durchmischung des Zweiphasensystems zur Erzeugung einer Dispersion;
f. Abtrennung von schwerer und leichter Phase in dem Zentrifugalextraktor;
g. Gewinnung des Zielproteins aus der leichten Phase.

Unter "Ankonzentrierung" ist in diesem Zusammenhang zu verstehen, dass das Zielprotein als Ergebnis in einer höheren Konzentration (Masse/Volumen) vorliegt. In der leichten Phase, aus der es nach dem Prozess gewonnen werden kann, liegt es in einer höheren Konzentration vor als in der Zellkultur oder dem Zellkulturüberstand bzw. in der schweren Phase, in der es in den Zentrifugalextraktor eingespeist wird.

Unter "Gewinnung des Zielproteins aus der leichte Phase" ist zu verstehen, dass die leichte Phase mit dem darin enthaltenen Zielprotein weiterverarbeitet wird, um das Zielprotein in der endgültig gewünschten Form zu erhalten. Dies kann weitere Reinigungsschritte, Ankonzentrierungsschritte, und/oder einen Wechsel des Mediums z.B. durch Umpufferung oder Abtrennung/Zusatz weiterer Substanzen beinhalten.

In einem Aspekt des Verfahrens enthält das Zweiphasen-System Polyethylenglykol mit einen Molekulargewicht zwischen 200 und 1000 g/mol.

In einem Aspekt des Verfahrens enthält das Zweiphasen-System Polyethylenglykol mit einen Molekulargewicht zwischen 400 und 600 g/mol.

In einem Aspekt des Verfahrens liegt das Polyethylenglykol im Zweiphasen-System in einer Konzentration von 18 bis 35 Gewichts-% vor.

In einem Aspekt des Verfahrens ist das Salz oder eines der Salze ein Phosphatsalz.

In einem Aspekt des Verfahrens beträgt der pH-Wert des Zweiphasensystems zwischen 5 und 7.

In einem Aspekt des Verfahrens wird der Zentrifugalextraktor mir schwerer Phase vorequilibiert.

In einem Aspekt des Verfahrens werden die beiden Phasen mit einer Zentrifugalbeschleunigung innerhalb eines Bereiches von 90 - 500 x g abgetrennt.

In einem Aspekt des Verfahrens werden die beiden Phasen mit einer Zentrifugalbeschleunigung innerhalb eines Bereiches von 90 - 450 x g abgetrennt.

In einem Aspekt des Verfahrens werden die beiden Phasen mit einer Zentrifugalbeschleunigung innerhalb eines Bereiches von 40 - 100 % der im verwendeten Gerät maximal möglichen Beschleunigung abgetrennt.

In einem Aspekt des Verfahrens beträgt das Dichteverhältnis der beiden Phasen mindestens 1.06.

### DETAILLIERTE BESCHREIBUNG DER ERFINDUNG

Wie oben ausgeführt, besteht ATPS aus einer wässrigen Lösung mit zwei unmischbaren Polymeren, bzw. einem Polymer und einem anorganischen Salz. Nach der Mischung der oben genannten Komponenten kommt es zur Ausbildung von zwei Phasen im System. Aufgrund unterschiedlicher biophysikalischer Eigenschaften erfolgt eine Verteilung der Biomoleküle in eine der beiden Phasen.

Zur Trennung von Wirtszellen, DNA und Proteinen aus Zellkultur mittels eines wässrigen Zwei-Phasen-Systems benötigt man geeignete Extraktionssysteme, bei dem sich die Wirtszellen und die DNA verstärkt in einer Phase anreichern und das Zielprotein in der jeweils anderen zweiten Phase. Je nach Proteinstabilität kann das Protein in der leichten und schweren Phase angereichert werden. Zur Trennung werden häufig PEG-Phosphatsysteme in unterschiedlichen Konzentrationen verwendet. Das PEG (Polyethylenglykol) reichert sich in der oberen leichten Phase an. Das Phosphat reichert sich in der schweren unteren Phase an. Proteine sind in PEG und Phosphat nur bedingt stabil und präzipitieren in hohen PEG- und Phosphatkonzentrationen. Da zur Ausbildung von Zwei-Phasen-Systemen hohe Konzentrationen benötigt werden, kann nicht jedes PEG und Phosphatsystem zur Trennung verwendet werden. PEG gibt es in verschiedenen Molekulargewichten. PEG mit Molekulargewichten > 1000 g/mol neigen dazu das Protein auch in geringen Konzentrationen zu präzipitieren. Für Extraktionen in denen sich das Protein in der leichten PEG Phase anreichern soll sind somit PEG mit einem Molekulargewicht < 1000 g/mol zu verwenden. Bei Verwendung von PEG mit einem solchen Molekulargewicht unter 1000 g/mol ist es auch nicht notwendig, dem Zweiphasenssystem ein zusätzliches Salz (z.B. Kochsalz) zuzufügen, um die Anreicherung in der leichten Phase zu erreichen, wie es im Stand der Technik beschrieben wird (16).

Da man zur Bildung von wässrigen Zwei-Phasen-Systemen mittels PEG400 (400 g/mol) und PEG600 (600 g/mol) hohe Konzentrationen von Phosphat benötigt, eignen sich solche Systeme besonders gut zur Prozessierung/Trennung in einem Zentrifugalextraktor.

Der Vorteil eines Zentrifugalextraktor im Gegensatz zu einer Bodenkolonne und einem Mixer Settler ist, dass er leichte und schwere Phase von stabilen wässrigen Extraktionssystemen in einigen Sekunden trennen kann. Dies im Gegensatz zur Sedimentation bei z.B. bei Mixer Settler Anlagen durch die Zentrifugalkraft ermöglicht. Um die schnellen Extraktionszeiten zu erhalten, ist die Auswahl von geeigneten Systemen notwendig. Die geringe Trennzeit von leichter und schwerer Phase ist in erster Linie vom hohen Dichteunterschied der leichten zu schweren Phase abhängig. Übergangszeiten von 2 - 10 s der Proteine von der schweren Phase in die leichte Phase lassen sich mit Dichteverhältnissen von leichter und schwerer Phase >= 1.18 ermöglichen.

In PEG400 und PEG600-Phosphatsystemen mit einem pH Bereich von 5 bis 7 sind die Proteine besonders stabil. Geeignet zur Trennung von Proteinen über wässrige Zwei-Phasen-Systeme bei hohen Ausbeuten sind Systeme mit folgenden Anteilen an PEG und Phosphat in der leichten Phase (PEG Phase).
PEG400/ Phosphat pH 5 bis 7:
   Mit 18 - 35 Gewichts-% PEG400 und 8 - 15 Gewichts% Phosphat
PEG600/ Phosphat pH 5 bis 7:
   Mit 18 - 35 Gewichts-% PEG600 und 6 - 15 Gewichts-% Phosphat

Die verwendeten PEG / Phosphatsysteme können durch Ihre Dichteverhältnisse >= 1.06 schnelle Prozesszeiten ermöglichen, bei zugleich hoher Proteinstabilität in der Aufnahmephase der PEG Phase.

Das jeweilige PEG/ Phosphatsystem wird eingewogen bzw. hergestellt. Dabei wird die Zellkultur in das wässrige Phasensystem gegeben. Über eine Rührapparatur wird das wässrige Phasensystem durchmischt. Als Phosphatsalze können beispielsweise Alkalihydrogenphosphate wie Natriumdihydrogenphosphat oder Kaliummonohydrogenphosphat verwendet werden. Der Zentrifugalextraktor wird vorteilhafterweise zuerst mit schwerer Phase equilbriert. Dabei kann der Rotor auf eine Zentrifugalbeschleunigung im Bereich von 40 - 100 % der maximal möglichen g-Kraft (je nach Gerät) eingestellt werden. Vorzugsweise kann eine Zentrifugalbeschleunigung von 90 - 450 x g angelegt werden, was einer Umdrehungszahl zwischen 2000 und 4500 U/min bei dem beispielhaften Zentrifugalextraktor Modell BXP040 der Firma Rousselet Robatel entspricht. Gleichzeitig wird über eine Pumpe die schwere Phase in den Zentrifugalextraktor gegeben. Wenn nur noch aus dem schweren-Phasen-Ablauf, die zugegebene schwere Phase ausströmt, kann anschließend das wässrige Phasengemisch mit der Zellkultur bei gleichem Pumpenfluss und Umdrehung zugegeben werden.

Leichte Phase (PEG Phase) mit dem Zielprotein wird nun über den Ablauf für die leichte Phase gewonnen, in der schweren Phase werden die Wirtszellen, DNA und deren Bruchstücke angereichert.

In einem anderen Aspekt der Erfindung kann zusätzlich zur Reinigung und Anreicherung des Zielproteins eine Ankonzentrierung des Zielproteins erreicht werden, also eine Erhöhung der Konzentration des Zielproteins in der leichten Phase gegenüber der Konzentration in der Zellkultur. Dafür kann eine weitere Prozessführung nach dem "mix & sep"-Prinzip angewandt werden. Dabei wird die Zellkultur in eine schwere Phase mit hoher Phosphatkonzentration und niedriger PEG Konzentration gegeben (z.B. 18 - 39 Gew. Prozent Phosphat und 10.5 - 0.2 Gew.% PEG). Eine leichte Phase (Proteinaufnahmephase) mit einer niedrigen Phosphat- und einer hohen PEG-Konzentration wird separat hergestellt (z.B. 2-10 Gew. Prozent Phosphat und 43.8 - 21.2 Gew.% PEG). Der Zentrifugalextraktor wird vorteilhafterweise zuerst mit schwerer Phase equilibriert, wie oben beschrieben. Wenn nur noch aus dem schweren-Phasen-Ablauf die zugegebene schwere Phase ausströmt kann, anschließend die leichte Phase über eine separate Pumpe in den Zentrifugalextraktor gegeben werden. Die schwere Phase (mit Zellkultur) wird bei hohen Flüssen (z.B. für den verwendeten Zentrifugalextraktor BXP040 6 - 25 kg/h) und die leichte Phase (Aufnahmephase) bei niedrigen Flüssen (z.B. für den verwendeten Zentrifugalextraktor BXP040 0.3 - 3 kg/h) in den Separator gegeben.

Das Verhältnis der Pumpenflüsse schwere Phase durch leichte Phase entspricht dabei dem Ankonzentrierungsverhältnis der Proteinlösung, und beträgt vorzugsweise das 5- bis 50fache (5 - 50 x). Im Zentrifugalextraktor kommt es in der Mischkammer (Zentrifugenbecher) zur Durchmischung von leichter und schwerer Phase.

Leichte Phase (PEG Phase) mit dem konzentrierten Zielprotein wird nun über den leichten Phase Ablauf gewonnen, in der schweren Phase werden die Wirtszellen, DNA und deren Bruchstücke angereichert. Durch den niedrigeren Pumpenfluss der leichten Phase ist das aus der Zentrifuge gewonnen Volumen der Proteinaufnahmephase geringer als das der schweren Phase und das Zielprotein entsprechend ankonzentriert.

### BEISPIELE

### Material und Methoden:

### Phosphatstammlösung:

Zur Herstellung 1 L einer 40 Gew. % Phoshatstammlösung werden 262.1 g Na-Dihydrogenphosphat (NaH₂PO₄ x 2 H₂O) sowie 198.4 g Kaliummonohydrogenphosphat K₂HPO₄) zu 539.5 g H₂O gegeben und homogen durchmischt.

### PEG400:

Polyethylenglykol mit einem mittleren MW von 400 g/mol

### Phasensystem A (zur Herstellung der Equilibrierungslösung)

Zur Herstellung von Phasensystem A wird PEG400 und Phosphatstammlösung sowie Natriumchlorid (NaCl) mit H₂O auf folgende Konzentrationen eingestellt. 23 Gew. % PEG400, 14 Gew. % Phosphat und 5 Gew. % NaCl, pH 6

### Phasensystem B mit Zellkultur

Zur Herstellung von Phasensystem B wird PEG400 und Phosphatstammlösung sowie Natriumchlorid (NaCl) mit einer Zellkultur auf folgende Konzentrationen eingestellt.

23 Gew. % PEG400, 14 Gew. % Phosphatstammlösung und 5 Gew. % NaCl, pH 6

### Phasensystem C (zur Herstellung der Equilibrierungslösung)

Zur Herstellung von Phasensystem A wird PEG400 und Phosphatstammlösung mit H₂0 auf folgende Konzentrationen eingestellt.

23 Gew. % PEG400, 14 Gew. % Phosphat, pH 6

### Phasensystem D mit Zellkultur

Zur Herstellung von Phasensystem D wird PEG400 und Phosphatstammlösung mit Zellkultur und H₂O auf folgende Konzentrationen eingestellt.

23 Gew. % PEG400, 14 Gew. % Phosphatstammlösung, pH 6

### Phasensystem E mit vorgereinigtem Protein

Zur Herstellung von Phasensystem E (schwere Phase) wird PEG400 und Phosphatstammlösung mit Protein und H₂O auf folgende Konzentrationen eingestellt. 1.25 Gew. % PEG400, 31 Gew. % Phosphatstammlösung, pH 6, und 3.3 Gew. % NaCl.

### Eukaryontische Zellkultur

- BIMAB19a. Die Zellkultur der CHO-Zellen mit Verunreinigungen wie DNA und Wirtszellen sowie Zielprotein wird nach mehreren Tagen Kultivierung erhalten. (Experiment 1)
- BIFAB39. Die Zellkultur der CHO-Zellen mit Verunreinigungen wie DNA und Wirtszellen sowie Zielprotein wird nach mehreren Tagen Kultivierung erhalten. (Experiment 3)

### Zellkulturüberstand

Der Zellkulturüberstand von CHO-Zellen mit Verunreinigungen wie DNA und Zielprotein wird nach mehreren Tagen Kultivierung durch Filtration oder Zentrifugation erhalten.

Die Konzentration von Zielprotein BIMAB01 wird mittels Protein A-Analytik auf 1.29 mg/ml bestimmt.

### Vorgereinigtes Protein

Das gereinigte Protein BIMAB01 liegt nach einem mehrstufigen Reinigungsverfahren (Affinitätschromatographie + Anionenaustauscher, Kationenautauscher, Filtration) vor.

### UV Bestimmung

Zur Bestimmung des Antikörper-Gehaltes von gereinigtem Protein wird auch eine UV Bestimmung bei 280 nm nach dem Lambert-Beer'schen Gesetz durchgeführt.

### Protein A HPLC

Zur Bestimmung des Antikörper-Gehaltes von Zellkulturfreiem Überstand sowie von gereinigtem Antiköper wurde eine Protein A HPLC an einer Waters oder Dionex-Anlage verwendet (Injektor Pumpen und Säulenofen W2790/5, UV-Detektor W2489). Der Antikörpergehalt der Lösungen wurde mittels des UV-Signals des sauer eluierenden Peaks bestimmt.

### Biacore Gehaltsbestimmung

Zur Bestimmung des Protein Gehalts von FAB39 dient die biospezifische Interaktionsanalyse am Biacore-System (BIA= Biospezifische / biomolekulare Interaktionsanalyse). Die Berechnung der Konzentration erfolgt relativ zu einer Standardkurve über die 4-parametrische Funktion der Biacore Control Software.

### Biacore Aktivitätsbestimmunq

Zur Bestimmung der Aktivität wird der Bindungsaktivität von FA39 an seinem Zielmolekül in Relation zum entsprechenden Referenzmaterial bestimmt. Hierzu dient die biospezifische Interaktionsanalyse am Biacore-System (BIA= Biospezifische / biomolekulare Interaktionsanalyse). Die Aktivität wird als Quotient aus aktiver Konzentration (Probe im Vergleich zum Referenzmaterial) und Gesamtproteingehalt (bestimmt mittels UV-Scan, Protein A/G - Biacore,
oder vergleichbar) in Prozent [%] berechnet.

### Dichte Messung

Zur Bestimmung der Dichte der leichten und schweren Phase wurde das Dichtemessgerät DM40 der Firma Mettler Toledo verwendet. Messungen wurden nach Herstellerangaben durchgeführt.

### Zellzahlbestimmung

Die Zellzahlbestimmung wurde mit einer Fuchs Rosenthal Zählkammer und dem Olympus BH2 Mikroskop durchgeführt. Dabei wurden die Zellen zuvor mit einer Trypanblaulösung angefärbt.

### Zentrifugalextraktor

Verwendet wurde das Modell BXP040 der Firma Rousselet Robatel mit einem Nutzvolumen von 0.11 L und einem Nenndurchsatz von 50 L/ h.

### Pumpe (Schlauchpumpe)

Watson Marlow 503S zur Speisung des Phasensystems und der Equlibirierungslösung

### Experimente:

### 1. Experiment: DNA und Zellabreicherung aus Zellkultur mittels Zentrifugalextraktor

Zur Separation von Wirtszellen, DNA und Zielprotein aus einer Zellkultur mittels wässriger Zwei-Phasen-Extraktion wird zuerst eine Phosphatstammlösung hergestellt. Danach wird das Phasensystem A durch Einwaage auf die oben genannten Konzentrationen eingestellt. In einem Scheidetrichter wird das Phasensystem A gegeben, zum Erhalt der Equilibrierungslösung des Zentrifugalextraktors (reine schwere Phase).

Nach dem sich zwei Phasen mit stabilen Volumen im Scheidetrichter ausgebildet haben, wird die leichte (obere) und schwere (untere) Phase (Equilibrierungslösung) getrennt und separat aufgefangen.

Zugleich wird das Phasensystem B mit Zellkultur hergestellt. Dieses wird mit einem Rührer homogen durchmischt.

Die Interphase (Durchmesser Schwere-Phase-Wehr - Durchmesser Leichte-Phase-Wehr) wurde auf 2 mm mittels Einbaues eines Schweren-Phasen-Wehrs reguliert.

Anschließend wird die Equilibrierungslösung mittels einer Pumpe bei hohem Fluss in den Zentrifugalextraktor über den Schweren-Phasen-Eingang gegeben, dies dient zur Equilibrierung des Zentrifugalextraktors. Dazu wird der Zentrifugalbecher auf eine Umdrehungszahl zwischen 2500 - 3500 RPM beschleunigt. Wenn nur noch aus den Schweren- Phasen-Ablauf des Zentrifugalextraktors, die zugegebene Equilibrierungslösung ausströmt, kann anschließend Phasensystem B (mit Zellkultur) bei gleichem Pumpenfluss und Umdrehungszahl über den Schweren-Phasen-Eingang eingespeist werden.

Leichte Phase (PEG Phase) mit dem Zielprotein wird nun über den Ablauf für die leichte Phase gewonnen, in der schweren Phase werden die Wirtszellen, DNA und deren Bruchstücke angereichert.

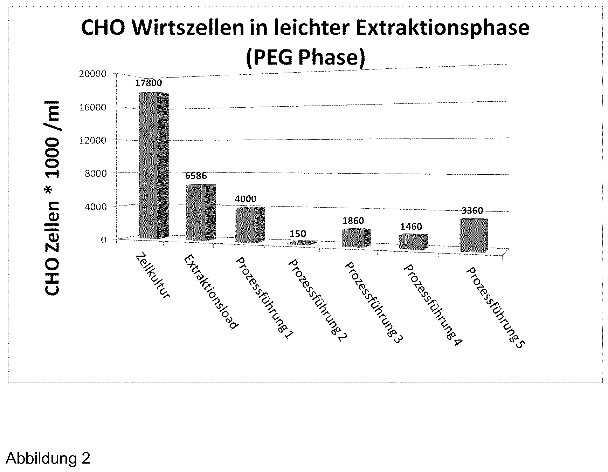

Abbildung 2 zeigt eine Messung der Zahl der CHO Wirtszellen in der leichten Phase nach Extraktion im Zentrifugalextraktor bei verschiedenen Prozessführungen.

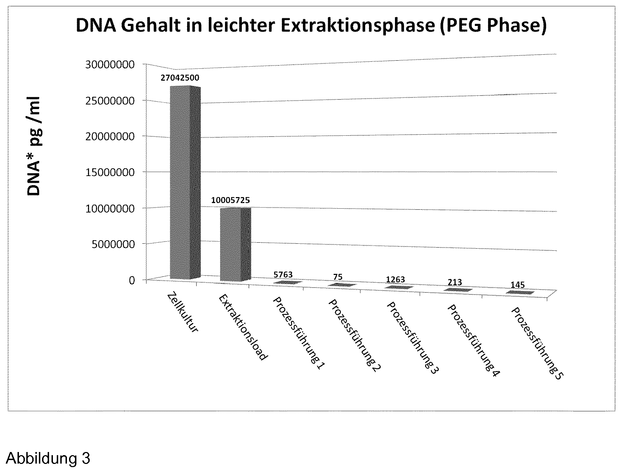

Abbildung 3 zeigt den DNA Gehalt in der leichten Phase nach Extraktion im Zentrifugalextraktor bei verschiedenen Prozessführungen.

**Tabelle 1: Prozessstrategien im Zentrifugalextraktor**

| Strategie | Pumpenfluss | Zentrifugenbecher Rotation |
|---|---|---|
| | L/H | RPM |
| Prozessführung 1 | 8.6 | 2500.0 |
| Prozessführung 2 | 22.8 | 2500.0 |
| Prozessführung 3 | 5.7 | 3500.0 |
| Prozessführung 4 | 22.8 | 3500.0 |
| Prozessführung 5 | 22.8 | 3000.0 |

### 2. Experiment: DNA Abreicherung aus Zellkulturüberstand mittels Zentrifugalextraktor

Zur Separation von DNA und Zielprotein aus einem Zellkulturüberstand mittels wässriger Zwei-Phasen-Extraktion wird zuerst eine Phosphatstammlösung hergestellt. Danach wird das Phasensystem A durch Einwaage auf die richtigen Konzentrationen wie oben beschrieben hergestellt. In einen Scheidetrichter wird das Phasensystem A gegeben. Nach dem sich zwei Phasen mit stabilen Volumen im Scheidetrichter ausgebildet haben, wird die leichte/obere und schwere/ untere Phase (Equilibrierungslösung) getrennt und separat aufgefangen.

Nach dem gleichen Vorgehen wird das Phasensystem B mit Zellkulturüberstand hergestellt. Dieses wird mit einem Rührer homogen durchmischt. Dabei werden 1750 g Phosphatstammlösung, 1150 g PEG400, 250 g NaCl und 1850 g Zellkulturüberstand eingewogen. Dadurch ergibt sich eine Protein Konzentration im Phasensystem B von 0.48 g/L sowie einer DNA Konzentration von 2941473 pg DNA /mg Protein.

Die Interphase im Zentrifugalextraktor (Durchmesser schwere Phase Wehr - Durchmesser leichte Phase Wehr) wird auf 2 mm mittels Einbaus eines schwere-Phasen-Wehrs reguliert. Anschließend werden 200 g der Equilibrierungslösung mittels einer Pumpe bei 22.8 L/H in den Zentrifugalextraktor über den schweren Phasen Eingang gepumpt. Dazu wird der Zentrifugalbecher auf eine Umdrehungszahl von 3000 RPM beschleunigt.

Wenn nur noch aus den schwere-Phase-Ablauf des Zentrifugalextraktors, die zugegebene Equilibrierungslösung ausströmt, werden 4077 ml Phasensystem B (mit Zellkulturüberstand) bei gleichem Pumpenfluss und Umdrehungszahl über den schwere-Phasen-Eingang eingespeist. Leichte Phase (PEG-Phase) mit dem Zielprotein wird nun über den leichten-Phase-Ablauf gewonnen. In der schweren Phase werden DNA und deren Bruchstücke angereichert.

**Tabelle 2: Ergebnisse zu Experiment 2**

| | |
|---|---|
| **Phasensystem B gesamt [ml]** | 4077 |
| **Konz. Protein in ATPS [mg/ml]** | 0.48 |
| **Protein gesamt [mg]** | 1945 |
| **DNA Gehalt gesamt ATPS [pg DNA /mg Protein]** | 2941473 |
| | |
| **Volumen leichte Phase [ml]** | 2573 |
| **Konz. MAB leichte Phase [mg/ml]** | 0.75 |
| **Protein in leichter Phase [mg]** | 1930 |
| **Ausbeute MAB leichte Phase [%]** | 99.2 |
| **DNA Gehalt leichte Phase [pg DNA /mg Protein]** | 144 |
| **DNA Abreicherung [log Stufe]** | 4.3 |
| | |
| **Volumen schwere Phase [ml]** | 1148 |
| **Konz. MAB schwere Phase [mg/ml]** | 0.01 |
| **Protein in schwerer Phase [mg]** | 11 |
| **DNA Gehalt [pg DNA /mg Protein]** | 401200000 |

### 3. Experiment: DNA und Zellabreicherung aus Zellkultur mittels Zentrifugalextraktor

Zur Separation von Wirtszellen, DNA und Zielprotein aus einer Zellkultur mittels wässriger Zwei-Phasen-Extraktion wird zuerst eine Phosphatstammlösung hergestellt. Danach wird das Phasensystem C durch Einwaage auf die oben genannten Konzentrationen eingestellt. In einem Scheidetrichter wird das Phasensystem C gegeben, zum Erhalt der Equilibrierungslösung des Zentrifugalextraktors (reine schwere Phase) und der Aufnehmerphase (Ankonzentrierungsphase - leichte Phase).

Nach dem sich zwei Phasen mit stabilen Volumen im Scheidetrichter ausgebildet haben, wird die leichte (obere) Phase (Ankonzentrierungsphase - leichte Phase) und schwere (untere) Phase (Equilibrierungslösung) getrennt und separat aufgefangen.

Zugleich wird das Phasensystem E mit gereinigtem Protein hergestellt. Dieses wird mit einem Rührer homogen durchmischt.

Die Interphase (Durchmesser Schwere-Phase-Wehr - Durchmesser Leichte-Phase-Wehr) wird auf 2 mm mittels Einbaues eines Schweren-Phasen-Wehrs reguliert.

Anschließend wird die Equilibrierungslösung mittels einer Pumpe bei hohem Fluss in den Zentrifugalextraktor über den Schweren-Phasen-Eingang gegeben, dies dient zur Equilibrierung des Zentrifugalextraktors. Dazu wird der Zentrifugalbecher auf eine Umdrehungszahl zwischen 2800 Umdrehungen pro Minute (RPM) beschleunigt. Wenn nur noch aus den Schweren- Phasen-Ablauf des Zentrifugalextraktors, die zugegebene Equilibrierungslösung ausströmt, kann anschließend Phasensystem D (mit Zellkultur) bei gleichem Pumpenfluss und Umdrehungszahl über den Schweren-Phasen-Eingang eingespeist werden.

Leichte Phase (PEG-Phase) mit dem Zielprotein wird nun über den Leichten-Phase-Ablauf gewonnen, in der schweren Phase werden die Wirtszellen, DNA und deren Bruchstücke angereichert.

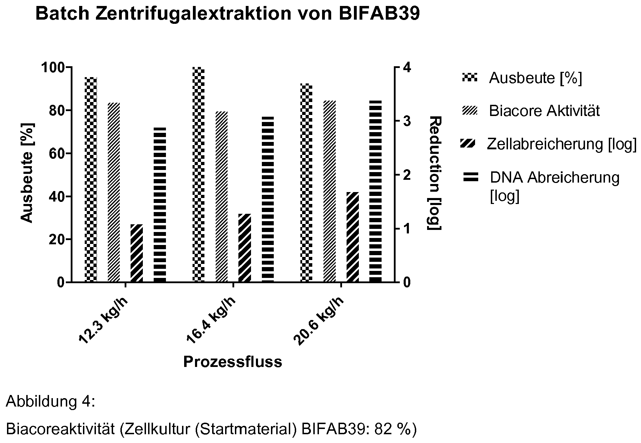

Abbildung 4 zeigt eine Messung der Zahl der CHO Wirtszellen, DNA Gehalt und der Biacore Aktivität in der leichten Phase nach Extraktion im Zentrifugalextraktor bei verschiedenen Prozessflüssen.

**Tabelle 3: Herstellung Phasensystem D**

| | |
|---|---|
| **ATPS System [g]** | 3000 |

| **Stammlösungen** | |
|---|---|
| **Phosphat pH 6%[w/w]** | 40 |
| **PEG400 %[w/w]** | 100 |

| **Proteingehalt Zellkultur BIFAB39** | |
|---|---|
| **[g/L]** | 1.53 |

| **System** | |
|---|---|
| **PEG400 %[w/w]** | 23 |
| **PO4 pH 6%[w/w]** | 14 |
| **NaCl %[w/w]** | 0 |
| **Proteingehalt System [g/L]** | 0,3 |

| **Einwaage** | |
|---|---|
| **Phosphat pH 6%[g]** | 1050 |
| **PEG400 %[g]** | 690 |
| **NaCl [g]** | 0 |
| **Zellkultur [g]** | 600 |
| **H₂O [g]** | 660 |

### 4. Experiment: Aufkonzentrierung von Protein (MAB) durch Prozessstrategie

Zur Aufkonzentrierung von Zielprotein kann der Zentrifugalextraktor mittels wässriger Zwei-Phasen-Extraktion benutzt werden. Zuerst wird eine Phosphatstammlösung hergestellt. Danach wird das Phasensystem A durch Einwaage auf die richtigen Konzentrationen wie oben beschrieben hergestellt. In einen Scheidetrichter wird das Phasensystem A mit der Equilibrierungslösung des Zentrifugalextraktors (reine schwere Phase) gegeben. Nach dem sich zwei Phasen mit stabilen Volumen im Scheidetrichter ausgebildet haben, wird die leichte/obere und schwere/ untere Phase (Equilibrierungslösung) getrennt und separat aufgefangen.

Nach dem gleichen Vorgehen wird das Phasensystem E mit gereinigtem Protein hergestellt (siehe Tabelle 5). Dieses wird mit einem Rührer homogen durchmischt.

**Tabelle 5: Herstellung Phasensystem E**

| | |
|---|---|
| **ATPS System [g]** | 5000 |

| **Stammlösungen** | |
|---|---|
| **Phosphat pH 6%[w/w]** | 40 |
| **PEG400 %[w/w]** | 100 |
| **Protein [g/L]** | 28.7 |

| **System** | |
|---|---|
| **PEG400 %[w/w]** | 31 |
| **PO4 pH 6%[w/w]** | 1.25 |
| **NaCl %[w/w]** | 3.3 |
| **Protein [g/L]** | 1 |

| **Einwaage** | |
|---|---|
| **Phosphat pH 6%[g]** | 3875 |
| **PEG400 %[g]** | 62.5 |
| **NaCl [g]** | 165 |
| **Protein [g]** | 174.22 |
| **H2O [g]** | 723.28 |

Die Interphase im Zentrifugalextraktor (Durchmesser schwere Phase Wehr - Durchmesser leichte Phase Wehr) wird auf 2 mm mittels Einbaus eines schwere-Phasen-Wehrs reguliert. Anschließend werden 500 g der Equilibrierungslösung mittels einer Pumpe bei 10 L/H (Liter pro Stunde) in den Zentrifugalextraktor über den schweren Phasen Eingang gepumpt. Dazu wird der Zentrifugalbecher auf eine Umdrehungszahl von 3000 RPM beschleunigt.

Wenn aus dem schwere-Phase-Ablauf des Zentrifugalextraktors nur noch die zugegebene Equilibrierungslösung ausströmt, wird nun die schwere Phase - Phasensystem E - über den schwere-Phasen-Eingang eingespeist und die leichte Phase (Aufnehmer-Phase) bei unterschiedlichen Pumpenflüssen (Prozessstrategien siehe Tabelle 6) über den leichten-Phasen-Eingang eingespeist. Leichte Phase (PEG-Phase) mit dem aufkonzentrierten Zielprotein wird nun über den leichten-Phase-Ablauf gewonnen.

**Tabelle 6: Prozessführung im Zentrifugalextraktor zur Ankonzentrierung (Mittels Fluss-Verhältnis von leichter und schwerer Phasen Pumpe)**

| Strategie | Pumpenfluss Schwere Phase | Pumpenfluss Leichte Phase | Fluss-Verhältnis schwerer zu leichter Phase |
|---|---|---|---|
| | Liter/Stunde | Liter/Stunde | |
| Prozessführung 1 | 4.3 | 4.3 | 1.0 |
| Prozessführung 2 | 10.0 | 4.3 | 2.3 |
| Prozessführung 3 | 20.0 | 4.3 | 4.7 |
| Prozessführung 4 | 13.1 | 1.4 | 9.4 |

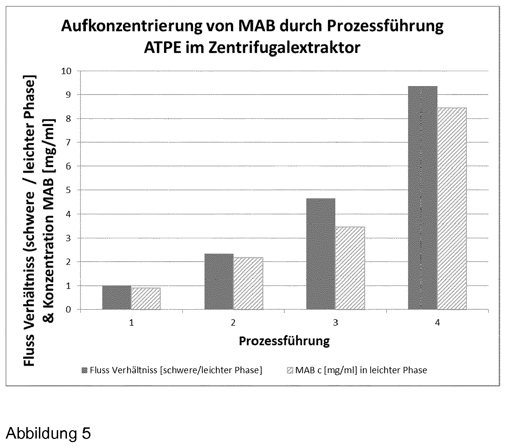

Ergebnisse siehe Abbildung 5.

### Definitionen von nicht SI Einheiten:

RPM= rounds per minute (Umdrehungen/min) von z.B. der Zentrifuge % w/w = Gew. %, Gewichtsprozent, Massenanteil

### LITERATURVERZEICHNIS

1. Wurm. Nature Biotechnology. 2004, 22 (1393-1398).
2. Liu et al. mAbs. mAbs 2:5, 480-499; September/October 2010; © 2010 Landes Bioscience, 2010, Volumne 2 Issue 5 (480-499).
3. Pramanik et al. Characterization of Impurities and Degradants Using Mass Spectrometry. 2011.
4. Walsh. Drug Discovery Today. 2010, Bd. 15, 17-18 (773-780).
5. Shukla et al. Biopharm International. 2008, Bd. Volume 21, Issue 5.
6. Labrou et al. J Biotechnol. 1994, Bd. 36, 2 (95-119).
7. Abhinav et al. Trends in Biotechnology. 2010, Bd. 28, 5 (253-261).
8. Chmiel. Bioprozesstechnik. Spektrum Akademischer Verlag. 2006.
9. Gottschalk et al. Process Scale Purification of Antibodies. 2008.
10. Brekke et al. Current Opinion in Pharmacology. 2003, 5 (544-550).
11. Voitl et al. Journal of Chromatography A. 2010, 1217 (5753-5760).
12. Bensch et al. Chemical Engineering Science. 2007, 7 (2011-2021).
13. Bora et al. Separation and Purification Technology. 2005, Bd. Volume 45, Issue 2 (153-156).
14. Kepka et al. Journal of Chromatography A. 2004, Bd. Volume 1057, Issues 1-2 (115-124).
15. Asenjo et al. Journal of Chromatography A. 1994, Bd. Volume 668, Issue 1 (129-137).
16. Azevedo et al. Trends in Biotechnology. 2009, Bd. Volume 27, Issue 4 (Pages 240-247).
17. Rosa et al. Journal of Biotechnology. 2009, Bd. Volume 139, Issue 4 (306-313).
18. Albertsons, P. Å. (1971) Partition of Cell Particles and Macromolecules, 2nd edn. Almqvist and Wiksell, Stockholm and Wiley, New York*.*
19. Oelmeier et Hubbuch, Journal of Chromatography A 2012, Bd. Volume 1252 (104-114*).*

## Patentansprüche

1. Verfahren zur selektiven Reinigung und Anreicherung von Immunglobulinen mittels eines wässrigen Zwei-Phasen-Systems mit den Schritten
a. Bereitstellen einer Zellkultur oder eines Zellkulturüberstandes, die das Zielprotein enthält;
b. Überführung der Zellkultur oder des Zellkulturüberstandes in ein wässriges Zweiphasensystem durch Zugabe von Polyethylenglykol mit einen Molekulargewicht zwischen 200 und 1000 g/mol, so dass das Polyethylenglykol in einer Konzentration von 18 bis 35 Gewichts-% vorliegt, und eines Phosphatsalzes;
c. Durchmischung des Zweiphasensystems zur Erzeugung einer Dispersion;
d. Abtrennung von schwerer und leichter Phase in einem Zentrifugalextraktor;
e. Gewinnung des Zielproteins aus der leichten Phase.

2. Verfahren zur selektiven Reinigung und Anreicherung und zusätzlichen Ankonzentrierung von Immunglobulinen mittels eines wässrigen Zwei-Phasen-Systems mit den Schritten
a. Bereitstellen einer Zellkultur oder eines Zellkulturüberstandes, die das Zielprotein enthält;
b. Bereitstellen einer leichten Phase des wässriges Zweiphasensystems durch Zugabe von Polyethylenglykol mit einen Molekulargewicht zwischen 200 und 1000 g/mol und eines Phosphatsalzes;
c. Überführen der Zellkultur oder des Zellkulturüberstandes in die schwere Phase des wässriges Zweiphasensystem durchs Zugabe von Polyethylenglykol mit einen Molekulargewicht zwischen 200 und 1000 g/mol und eines Phosphatsalzes;
d. Separate Zuführung von leichter und schwere Phase in die Mischkammer des Zentrifugalextraktors mit unterschiedlichen Flussraten, wobei das Verhältnis der Flussraten dem gewünschten Ankonzentrierungsverhältnis entspricht;
e. Durchmischung des Zweiphasensystems zur Erzeugung einer Dispersion;
f. Abtrennung von schwerer und leichter Phase in dem Zentrifugalextraktor;
g. Gewinnung des Zielproteins aus der leichten Phase.

3. Verfahren nach einem der vorangehenden Ansprüche, **dadurch gekennzeichnet, dass** das Polyethylenglykol ein Molekulargewicht zwischen 400 und 600 g/mol hat.

4. Verfahren nach einem der vorangehenden Ansprüche, **dadurch gekennzeichnet, dass** der pH-Wert des Zweiphasensystems zwischen 5 und 7 beträgt.

5. Verfahren nach einem der vorangehenden Ansprüche, **dadurch gekennzeichnet, dass** der Zentrifugalextraktor mir schwerer Phase vorequilibiert wird.

6. Verfahren nach einem der vorangehenden Ansprüche, **dadurch gekennzeichnet, dass** die beiden Phasen mit einer Zentrifugalbeschleunigung von 90 - 500 x g abgetrennt werden.

7. Verfahren nach einem der vorangehenden Ansprüche, **dadurch gekennzeichnet, dass** das Dichteverhältnis der beiden Phasen mindestens 1.06 beträgt.

## Claims

1. Method for the selective purification and concentration of immunoglobulins by means of an aqueous two-phase system, comprising the steps of:
a. preparing a cell culture or a cell culture supernatant which contains the target protein;
b. converting the cell culture or the cell culture supernatant into an aqueous two-phase system by the addition of polyethylene glycol having a molecular weight between 200 and 1000 g/mol such that the polyethylene glycol is present in a concentration of from 18 to 35 wt.%, and a phosphate salt;
c. thoroughly mixing the two-phase system to produce a dispersion;
d. separating the heavy and light phase in a centrifugal extractor;
e. recovering the target protein from the light phase.

2. Method for the selective purification and accumulation and additional concentration of immunoglobulins by means of an aqueous two-phase system, comprising the steps of:
a. preparing a cell culture or a cell culture supernatant which contains the target protein;
b. providing a light phase of the aqueous two-phase system by adding polyethylene glycol having a molecular weight between 200 and 1000 g/mol and a phosphate salt;
c. converting the cell culture or the cell culture supernatant into the heavy phase of the aqueous two-phase system by the addition of polyethylene glycol having a molecular weight between 200 and 1000 g/mol and a phosphate salt;
d. separately feeding the light and heavy phases into the mixing chamber of the centrifugal extractor at different flow rates, wherein the ratio of the flow rates corresponds to the desired concentration ratio;
e. thoroughly mixing the two-phase system to produce a dispersion;
f. separating the heavy and light phase in the centrifugal extractor;
g. recovering the target protein from the light phase.

3. Method according to either of the preceding claims, **characterised in that** the polyethylene glycol has a molecular weight of between 400 and 600 g/mol.

4. Method according to any one of the preceding claims, **characterised in that** the pH of the two-phase system is between 5 and 7.

5. Method according to any one of the preceding claims, **characterised in that** the centrifugal extractor is pre-equilibrated with the heavy phase.

6. Method according to any one of the preceding claims, **characterised in that** the two phases are separated with a centrifugal acceleration of 90 - 500 x g.

7. Method according to any one of the preceding claims, **characterised in that** the density ratio of the two phases is at least 1.06.

## Revendications

1. Procédé de purification sélective et d'enrichissement d'immunoglobulines au moyen d'un système aqueux à deux phases, comportant les étapes de
a. mise à disposition d'une culture cellulaire ou d'un surnageant de culture cellulaire qui contient la protéine cible ;
b. transfert de la culture cellulaire ou du surnageant de culture cellulaire dans un système aqueux à deux phases par addition de polyéthylène glycol ayant un poids moléculaire entre 200 et 1000 g/mole, de sorte que le polyéthylène glycol soit en une concentration de 18 à 35 % en poids, et d'un sel de phosphate ;
c. mélange du système à deux phases pour produire une dispersion ;
d. séparation de la phase lourde et de la phase légère dans un extracteur centrifuge ;
e. obtention de la protéine cible de la phase légère.

2. Procédé de purification sélective et de concentration supplémentaire d'immunoglobulines au moyen d'un système aqueux à deux phases comportant les étapes de
a. mise à disposition d'une culture cellulaire ou d'un surnageant de culture cellulaire qui contient la protéine cible ;
b. mise à disposition d'une phase légère du système aqueux à deux phases par addition de polyéthylène glycol ayant un poids moléculaire entre 200 et 1000 g/mole, et d'un sel de phosphate ;
c. transfert de la culture cellulaire ou du surnageant de culture cellulaire dans la phase lourde mélange du système aqueux à deux phases par addition de polyéthylène glycol ayant un poids moléculaire entre 200 et 1000 g/mole et d'un sel de phosphate ;
d. apport séparé de phase légère et de phase lourde dans la chambre de mélange de l'extracteur centrifuge avec des débits différents, dans lequel le rapport des débits correspond au rapport de concentration souhaité ;
e. mélange du système à deux phases pour produire une dispersion ;
f. séparation de la phase lourde et de la phase légère dans un extracteur centrifuge ;
g. obtention de la protéine cible de la phase légère.

3. Procédé selon l'une des revendications précédentes, **caractérisé en ce que** le polyéthylène glycol a un poids moléculaire entre 400 et 600 g/mole.

4. Procédé selon l'une des revendications précédentes, **caractérisé en ce que** le pH du système à deux phases se situe entre 5 et 7.

5. Procédé selon l'une des revendications précédentes, **caractérisé en ce que** l'extracteur centrifuge est pré-équilibré avec la phase lourde.

6. Procédé selon l'une des revendications précédentes, **caractérisé en ce que** les deux phases sont séparées avec une vitesse de centrifugation de 90 à 500 x g.

7. Procédé selon l'une des revendications précédentes, **caractérisé en ce que** le rapport de densité des deux phases est d'au moins 1,06.
